# EUROPEAN PATENT APPLICATION

(11) **EP 3 355 051 A1**
(43) Date of publication of application: **01.08.2018**
(21) Application number: 17789062.1
(22) Date of filing: 28.02.2017
(51) Int. Cl.: G01N 27/06, G01N 27/22, G01R 27/22, G01R 27/26

(54) **SENSOR AND METHOD FOR PRODUCING SENSOR**

(30) Priority: 27.04.2016 JP 2016089096
(71) Applicant: KYB Corporation, Tokyo 105-6111 (JP)
(72) Inventor: YOSHIDA, Takahiro, Tokyo 105-6111 (JP); KAMEDA, Yukinori, Tokyo 105-6111 (JP)
(74) Representative: Horn Kleimann Waitzhofer Patentanwälte PartG mbB
(86) International application number: PCT/JP2017/007849
(87) International publication number: WO 2017/187773

(57) **Abstract**

A sensor that detects the state of a fluid more appropriately is provided. A sensor (1) detects the state of a fluid. This sensor (1) includes an electrode (21), an electrode (22), a first opposing-side resin portion (31A), and a second opposing-side resin portion (31B). The electrode (21) and the electrode (22) are arranged parallel to each other. The first opposing-side resin portion (31A) covers an area of a peripheral face of the electrode (21), the area opposing the electrode (22). The second opposing-side resin portion (31B) covers an area of a peripheral face of the electrode (22), the area opposing the electrode (21).

## Description

### Technical Field

The present invention relates to a sensor and a method for manufacturing a sensor.

### Background Art

Techniques for detecting the state of a fluid have been proposed (e.g. see Patent Document 1).

Patent Document 1 describes an apparatus for detecting the state of oil that is circulated and supplied to rotary and sliding portions in order to smoothly operate these parts while preventing abrasion thereof. This apparatus includes two plates, which are installed parallel to each other in an oil passage. The apparatus obtains the permittivity and conductivity of the oil by applying an AC voltage across these two plates, and detects the state of the oil based on the obtained permittivity and conductivity.

### Citation List

### Patent Literature

[Patent Document 1]
Japanese Patent Application Laid Open No. 2009-2693

### Summary of Invention

### Technical Problem

There are cases where foreign substances, such as pieces of metal, are mixed into the oil described in Patent Document 1 due to abrasion of the rotary and sliding portions. If a foreign substance is attached to the opposing faces of the two plates, an error may occur in the measurement of the permittivity and conductivity of the oil. There is a concern that the permittivity and conductivity of the oil cannot be correctly obtained, resulting in the inability to appropriately detect the state of the oil.

The present invention has been made in view of the foregoing problem, and provides a sensor for appropriately detecting the state of a fluid.

### Solution to Problem

In one or more embodiments of the present invention, a sensor for detecting a state of a fluid includes: a first electrode and a second electrode that are arranged parallel to each other; a first opposing-side resin portion that covers an area of a peripheral face of the first electrode, the area opposing the second electrode; and a second opposing-side resin portion that covers an area of a peripheral face of the second electrode, the area opposing the first electrode, wherein the first opposing-side resin portion and the second opposing-side resin portion are spaced apart from each other.

### Brief Description of the Drawings

FIG. 1 is a block diagram of a state detection apparatus that includes a sensor according to a first embodiment of the present invention.
FIG. 2 is a perspective view of the sensor according to the first embodiment of the present invention.
FIG. 3 is a top view of the sensor according to the first embodiment of the present invention.
FIG. 4 is a perspective view of a sensor according to a second embodiment of the present invention.
FIG. 5 is a top view of the sensor according to the second embodiment of the present invention.
FIG. 6 is a perspective view of a sensor according to a third embodiment of the present invention.
FIG. 7 is a top view of the sensor according to the third embodiment of the present invention.
FIG. 8 is a top view of a sensor according to a fourth embodiment of the present invention.

### Description of Embodiments

Hereinafter, the embodiments of the present invention will be described with reference to the drawings. Note that the structures in the following embodiments may be replaced with existing structures as appropriate. Also, different variations thereof including combinations with other existing structures are possible. Accordingly, the following embodiments are not intended to limit the content of the invention described in the claims. Although an XYZ orthogonal coordinate system is provided in the following diagrams for ease of description and understanding, it is merely set for the purpose of consistency in the directions in the drawings throughout the description and does not indicate absolute coordinates.

### First Embodiment

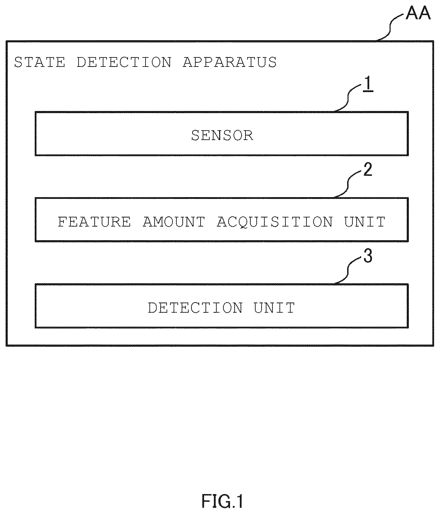

FIG. 1 is a block diagram of a state detection apparatus AA, which includes a sensor 1 according to the first embodiment of the present invention. The state detection apparatus AA includes the sensor 1, a feature amount acquisition unit 2, and a detection unit 3, and detects the state of a lubricating oil.

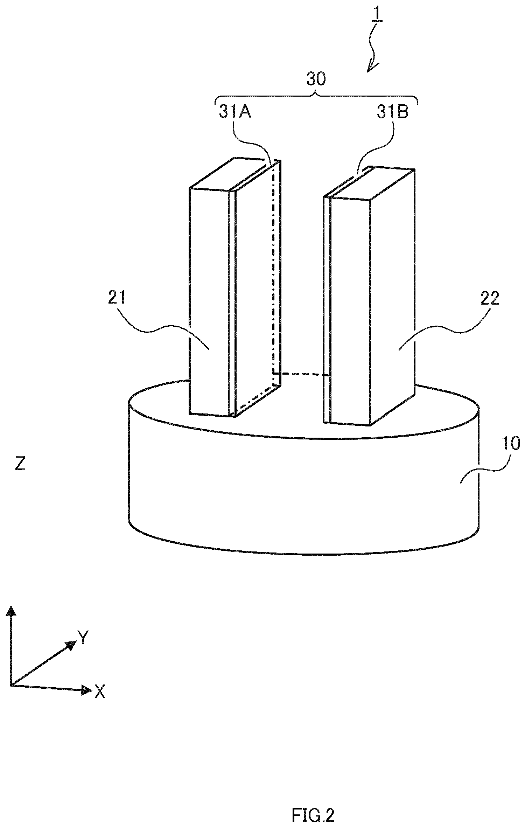

FIG. 2 is a perspective view of the sensor 1. The sensor 1 includes a columnar base portion 10, a pair of electrodes 21 and 22, which are installed upright on the base portion 10, and a resin portion 30.

The electrodes 21 and 22 are flat plates and are arranged parallel to each other. The electrodes 21 and 22 are installed upright on the base portion 10 with the base end side (negative Z-side) of the electrodes 21 and 22 embedded in the base portion 10.

The electrodes 21 and 22 are installed in a lubricating oil. The feature amount acquisition unit 2 in FIG. 1 applies an AC voltage across the electrodes 21 and 22 installed in the lubricating oil, and obtains the permittivity and conductivity of the lubricating oil between the electrodes 21 and 22. The detection unit 3 in FIG. 1 detects the state of the lubricating oil based on the permittivity and conductivity of the lubricating oil that are obtained by the feature amount acquisition unit 2.

The feature amount acquisition unit 2 and the detection unit 3 are realized using a CPU, a memory (RAM), a hard disk, and the like.

The hard disk stores an operating system, a program for executing a series of processes for detecting the state of the lubricating oil, and the like. Note that the hard disk need only be a non-temporary recording medium, and may also be, for example, a nonvolatile memory such as an EPROM or a flash memory, a CD-ROM, or the like.

The CPU uses the memory to read out data and programs stored in the hard disk as appropriate and computes and executes the same as appropriate.

The resin portion 30 is made of non-conductive resin, and is integrated with the electrodes 21 and 22 by means of insertion molding. The non-conductive resin for forming the resin portion 30 may be, for example, a polybutylene terephthalate (PBT) resin, a liquid crystal polymer (LCP) resin, a nylon 66 (PA66) resin, a fluoropolymer, or the like.

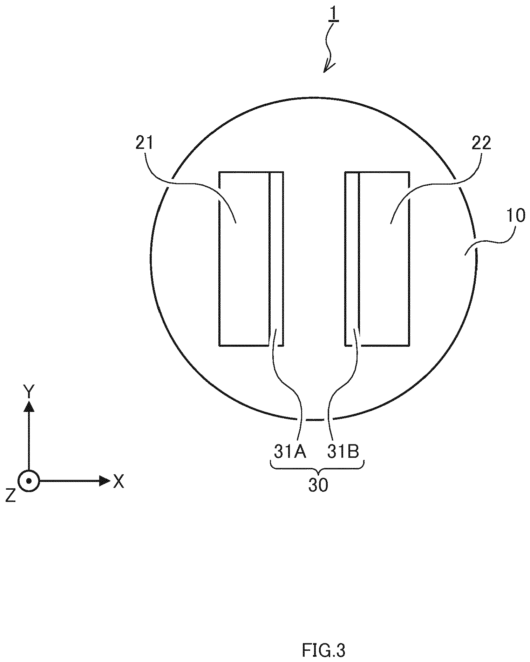

The resin portion 30 includes a first opposing-side resin portion 31A and a second opposing-side resin portion 31B. The first opposing-side resin portion 31A and the second opposing-side resin portion 31B will be described using FIG. 3.

FIG. 3 is a top view of the sensor 1. The first opposing-side resin portion 31A covers a peripheral face (the face on the positive X-side) of a portion of the electrode 21 that is exposed from the base portion 10, the peripheral face opposing the electrode 22. The second opposing-side resin portion 31B covers a peripheral face (the face on the negative X-side) of a portion of the electrode 22 that is exposed from the base portion 10, the peripheral face opposing the electrode 21. The first opposing-side resin portion 31A and the second opposing-side resin portion 31B are spaced apart from each other. For this reason, upon the electrodes 21 and 22 being installed in the lubricating oil, the lubricating oil flows between the first opposing-side resin portion 31A and the second opposing-side resin portion 31B, and there is lubricating oil present between the electrodes 21 and 22.

As described above, in the sensor 1, the first opposing-side resin portion 31A covers the peripheral face of the portion of the electrode 21 that is exposed from the base portion 10, the peripheral face opposing the electrode 22. As a result, for example, even if a foreign substance such as varnish or sludge is included in the lubricating oil due to contamination, the foreign substance is not directly attached to the peripheral face of the electrode 21 that opposes the electrode 22. Also, in the sensor 1, the second opposing-side resin portion 31B covers the peripheral face of the portion of the electrode 22 that is exposed from the base portion 10, the peripheral face opposing the electrode 21. As a result, even if a foreign substance is included in the lubricating oil, the foreign substance is not directly attached to the peripheral face of the electrode 22 that opposes the electrode 21. With the above-described configuration, in the sensor 1, the aforementioned foreign substance is not directly attached to the peripheral face of the electrode 21 that opposes the electrode 22 and the peripheral face of the electrode 22 that opposes the electrode 21. As a result, the permittivity and conductivity of the lubricating oil can be correctly obtained while preventing an error in the measurement of the permittivity and conductivity of the lubricating oil from occurring due to direct attachment of a foreign substance, and the state of the lubricating oil can be detected more appropriately. If a foreign substance is directly attached to the peripheral faces of the electrodes 21 and 22, there is a concern that the electrodes 21 and 22 corrode. However, in the sensor 1, a foreign substance is not directly attached to the peripheral face of the electrode 21 that opposes the electrode 22 and the peripheral face of the electrode 22 that opposes the electrode 21, as mentioned above. As a result, corrosion of the electrodes 21 and 22 can be suppressed. In addition, the resin used in the first opposing-side resin portion 31A and the second opposing-side resin portion 31B bonds less with other substances than the metal used in the electrodes, due to having a stable molecular structure. For this reason, in the sensor 1, it is possible to reduce the possibility that a foreign substance is attached to the electrode 21 via the first opposing-side resin portion 31A or to the electrode 22 via the second opposing-side resin portion 31B.

If a vibration of an apparatus to which the sensor 1 is attached or a vibration occurring around this apparatus is propagated to the electrodes 21 and 22, the electrodes 21 and 22 may bend. If the electrode 21 bends in the thickness direction (X direction) of the electrode 21, or if the electrode 22 bends in the thickness direction (X direction) of the electrode 22, there is a concern that the amount of the lubricating oil present between the electrodes 21 and 22 changes, and the permittivity and conductivity of the lubricating oil cannot be correctly obtained. In the sensor 1, the first opposing-side resin portion 31A covers the peripheral face of the portion of the electrode 21 that is exposed from the base portion 10, the peripheral face opposing the electrode 22. As a result, the rigidity of the electrode 21 in the thickness direction of the electrode 21 increases. Also, in the sensor 1, the second opposing-side resin portion 31B covers the peripheral face of the portion of the electrode 22 that is exposed from the base portion 10, the peripheral face opposing the electrode 21. As a result, the rigidity of the electrode 22 in the thickness direction of the electrode 22 increases. With the above-described configuration, in the sensor 1, the bending of the electrode 21 in the thickness direction of the electrode 21 and the bending of the electrode 22 in the thickness direction of the electrode 22 can be suppressed even if the aforementioned vibration is propagated to the electrodes 21 and 22. Thus, a change in the amount of the lubricating oil present between the electrodes 21 and 22 can be suppressed, and the permittivity and conductivity of the lubricating oil can be obtained more correctly.

The bending of the electrodes 21 and 22 due to the aforementioned vibration is more likely to occur the larger the electrodes 21 and 22 are. However, in the sensor 1, the rigidity of the electrode 21 in the thickness direction of the electrode 21 is increased by the first opposing-side resin portion 31A as mentioned above, and the rigidity of the electrode 22 in the thickness direction of the electrode 22 is increased by the second opposing-side resin portion 31B as mentioned above. Thus, the electrodes 21 and 22 can be made larger while suppressing the bending of the electrodes 21 and 22 due to the aforementioned vibration. Accordingly, the amount of the lubricating oil present between the electrodes 21 and 22 can be increased by expanding the area of the opposing faces of the electrodes 21 and 22, and it is therefore possible to more correctly obtain the permittivity and conductivity of the lubricating oil.

In the sensor 1, the first opposing-side resin portion 31A and the second opposing-side resin portion 31B are spaced apart from each other. For this reason, upon the electrodes 21 and 22 being installed in the lubricating oil, the lubricating oil flows between the first opposing-side resin portion 31A and the second opposing-side resin portion 31B, and the lubricating oil is present between the electrodes 21 and 22. Accordingly, the permittivity and conductivity of the lubricating oil between the electrodes 21 and 22 can be obtained by applying an AC voltage across the electrodes 21 and 22.

In the sensor 1, the electrodes 21 and 22 are covered with the resin portion 30 by means of insertion molding, and the resin portion 30 is integrally formed with the base portion 10. With this configuration, a process of covering the electrodes 21 and 22 with the resin portion 30 can be shortened.

Note that, in this embodiment, the resin portion 30 is made of a non-conductive resin. However, the present invention is not limited thereto, and the resin portion 30 may also be made of a conductive resin.

In this embodiment, the sensor 1 obtains the permittivity and conductivity of the lubricating oil, and detects the state of the lubricating oil based on the obtained permittivity and conductivity. However, the present invention is not limited thereto, and the sensor 1 may also obtain either one of the permittivity and conductivity of the lubricating oil, and detect the state of lubricating oil based on the obtained result. Alternatively, the sensor 1 may also obtain an electrical characteristic of the lubricating oil other than the permittivity and conductivity (e.g. capacitance, dissipation factor (tanδ), characteristic impedance Z etc.), and detect the state of the lubricating oil based on the obtained electrical characteristic.

In this embodiment, the sensor 1 detects the state of the lubricating oil. However, the present invention is not limited thereto, and the state of any kind of fluid can be detected. For example, the sensor 1 can also detect the degree of contamination of water, and can also detect the quality of a chemical agent or a beverage.

In this embodiment, the electrodes 21 and 22 are formed with flat plates, and these electrodes 21 and 22 are arranged parallel to each other. However, the present invention is not limited thereto, and the electrodes 21 and 22 may also be formed with columnar or ellipse plates, for example. Also, for example, a configuration may also be employed in which the electrodes 21 and 22 are formed with two cylindrical plates having different inner diameters, as in the case of a coaxial cylindrical capacitor, and either one of the electrodes 21 and 22 is arranged within the other one with the center axes thereof matching each other. In this case, an area of the peripheral face of the portion of the electrode 21 that is exposed from the base portion 10, the area opposing the electrode 22, may be covered by the first opposing-side resin portion 31A, and an area of the peripheral face of the portion of the electrode 22 that is exposed from the base portion 10, the area opposing the electrode 21, may be covered by the second opposing-side resin portion 31B.

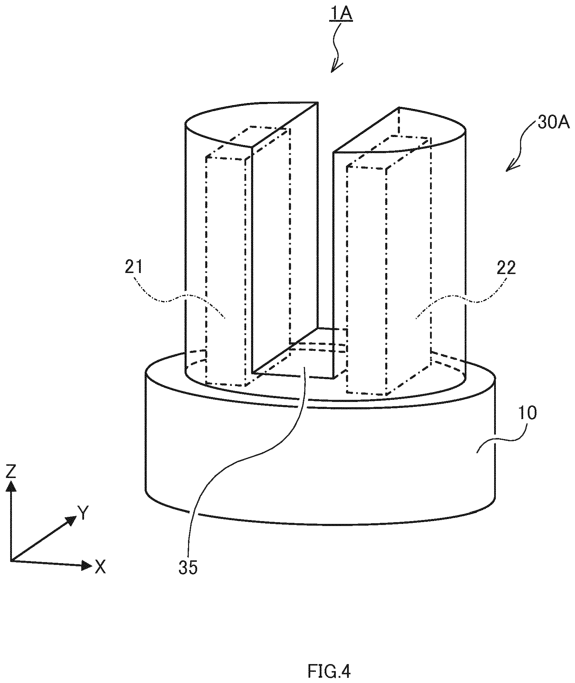

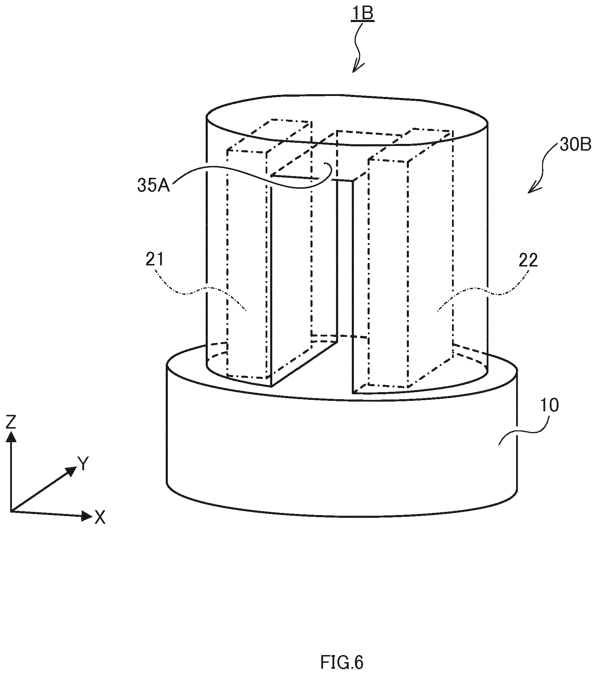

In this embodiment, the first opposing-side resin portion 31A and the second opposing-side resin portion 31B may also be connected to each other using a connecting resin portion 35, which will be described later using FIGS. 4, 5, and 7, or using a connecting resin portion 35A, which will be described later using FIG. 6. By connecting the first opposing-side resin portion 31A and the second opposing-side resin portion 31B, the bending of the electrodes 21 and 22 due to the aforementioned vibration can be further suppressed.

### Second Embodiment

FIG. 4 is a perspective view of a sensor 1A according to the second embodiment of the present invention. The sensor 1A is provided, in place of the sensor 1 according to the first embodiment of the present invention shown in FIGS. 2 and 3, in the state detection apparatus AA in FIG. 1. This sensor 1A differs from the sensor 1 in that it includes a resin portion 30A in place of the resin portion 30, and is the same as the sensor 1 regarding the other portions. Accordingly, the same portions as those in the first embodiment are assigned the same reference numerals, and descriptions thereof are omitted.

The resin portion 30A is made of a non-conductive resin, and is integrated with the electrodes 21 and 22 by means of insertion molding.

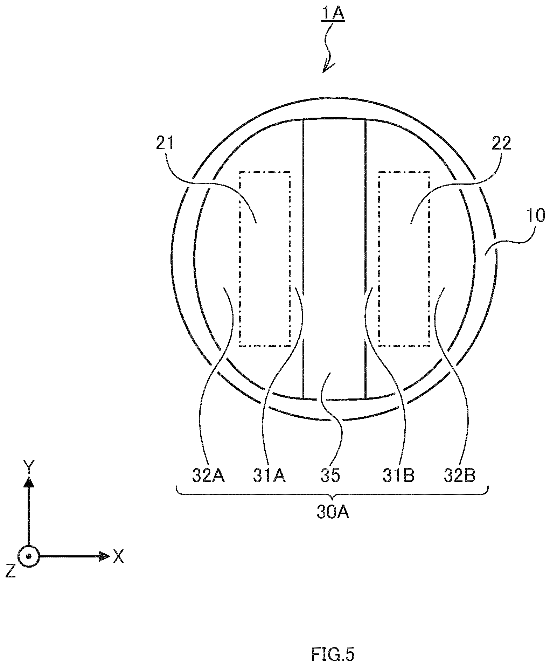

FIG. 5 is a top view of the sensor 1A. The resin portion 30A includes a first back-side resin portion 32A, a second back-side resin portion 32B, and a connecting resin portion 35, in addition to the first opposing-side resin portion 31A and the second opposing-side resin portion 31B.

The first back-side resin portion 32A covers a peripheral face of the portion of the electrode 21 that is exposed from the base portion 10, excluding the face covered by the first opposing-side resin portion 31A. For this reason, the peripheral face of the portion of the electrode 21 that is exposed from the base portion 10 on the side opposite to the face opposing the electrode 22 is covered by the first back-side resin portion 32A.

The second back-side resin portion 32B covers a peripheral face of the portion of the electrode 22 that is exposed from the base portion 10, excluding the face covered by the second opposing-side resin portion 31B. For this reason, the peripheral face of the portion of the electrode 22 that is exposed from the base portion 10 on the side opposite to the face opposing the electrode 21 is covered by the second back-side resin portion 32B.

The connecting resin portion 35 is in contact with the base portion 10, and connects the first opposing-side resin portion 31A and the second opposing-side resin portion 31B to each other on the base end side of the electrodes 21 and 22.

As described above, the sensor 1A includes the first opposing-side resin portion 31A and the second opposing-side resin portion 31B, and the electrodes 21 and 22 are covered by the resin portion 30A by means of insertion molding, similarly to the sensor 1 according to the first embodiment of the present invention. Thus, the sensor 1A can achieve the same effect as that of the sensor 1 according to the first embodiment of the present invention.

In the sensor 1A, the first back-side resin portion 32A covers the peripheral face of the portion of the electrode 21 that is exposed from the base portion 10 on the side opposite to the face opposing the electrode 22. This configuration further increases the rigidity of the electrode 21 in the thickness direction of the electrode 21. Also, in the sensor 1A, the second back-side resin portion 32B covers the peripheral face of the portion of the electrode 22 that is exposed from the base portion 10 on the side opposite to the face opposing the electrode 21. This configuration further increases the rigidity of the electrode 22 in the thickness direction of the electrode 22. With the above-described configuration, the sensor 1A can further suppress a change in the amount of the lubricating oil present between the electrodes 21 and 22, and can more correctly obtain the permittivity and conductivity of the lubricating oil.

In the sensor 1A, the rigidity of the electrode 21 in the thickness direction of the electrode 21 is increased using the first back-side resin portion 32A, as mentioned above. For this reason, the rigidity of the electrode 21 in the thickness direction of the electrode 21 can be increased using not only the first opposing-side resin portion 31A but also the first back-side resin portion 32A. Accordingly, the first opposing-side resin portion 31A can be made thinner. In the sensor 1A, the rigidity of the electrode 22 in the thickness direction of the electrode 22 is increased using the second back-side resin portion 32B, as mentioned above. For this reason, the rigidity of the electrode 22 in the thickness direction of the electrode 22 can be increased using not only the second opposing-side resin portion 31B but also the second back-side resin portion 32B. Accordingly, the second opposing-side resin portion 31B can be made thinner. With the above-described configuration, the first opposing-side resin portion 31A and the second opposing-side resin portion 31B can be made thinner while achieving an increase in the rigidity of the electrode 21 in the thickness direction of the electrode 21 and an increase in the rigidity of the electrode 22 in the thickness direction of the electrode 22. Thus, the amount of the lubricating oil present between the electrodes 21 and 22 can be increased, and it is therefore possible to more correctly obtain the permittivity and conductivity of the lubricating oil.

In the sensor 1A, the rigidity of the electrode 21 in the thickness direction of the electrode 21 is increased using the first back-side resin portion 32A as mentioned above. Also, the rigidity of the electrode 22 in the thickness direction of the electrode 22 is increased using the second back-side resin portion 32B as mentioned above. As a result, the electrodes 21 and 22 can be made larger while suppressing the bending of the electrodes 21 and 22 due to a vibration of the apparatus to which the sensor 1A is attached and a vibration occurring around this apparatus. Accordingly, the amount of the lubricating oil present between the electrodes 21 and 22 can be increased by expanding the area of the opposing faces of the electrodes 21 and 22, and it is therefore possible to more correctly obtain the permittivity and conductivity of the lubricating oil.

In the sensor 1A, the first opposing-side resin portion 31A and the second opposing-side resin portion 31B are connected to each other on the base end side of the electrodes 21 and 22, using the connecting resin portion 35. Thus, the bending of the electrodes 21 and 22 due to the aforementioned vibration can be further suppressed.

In the sensor 1A, the resin portion 30A is made of a non-conductive resin. For this reason, although the first opposing-side resin portion 31A and the second opposing-side resin portion 31B are connected to each other using the connecting resin portion 35, there is no conduction between the first opposing-side resin portion 31A and the second opposing-side resin portion 31B. Accordingly, an AC voltage can be applied across the electrodes 21 and 22.

Although, in this embodiment, the resin portion 30A is made of a non-conductive resin, the present invention is not limited thereto, and the resin portion 30A may also be made of a conductive resin. However, in the case of making the resin portion 30A using a conductive resin, a configuration in which the connecting resin portion 35 is not provided in the resin portion 30A needs to be employed.

In this embodiment, the sensor 1A obtains the permittivity and conductivity of the lubricating oil, and detects the state of the lubricating oil based on the obtained permittivity and conductivity. However, the present invention is not limited thereto, and the sensor 1A may also obtain either one of the permittivity and conductivity of the lubricating oil, and detect the state of the lubricating oil based on the obtained result. Alternatively, the sensor 1A may also obtain an electrical characteristic of the lubricating oil other than the permittivity and conductivity (e.g. capacitance, dissipation factor (tanδ), characteristic impedance Z etc.), and detect the state of the lubricating oil based on the obtained electrical characteristic.

In this embodiment, the sensor 1A detects the state of the lubricating oil. However, the present invention is not limited thereto, and the state of any kind of fluid can be detected. For example, the sensor 1A can also detect the degree of contamination of water, and can also detect the quality of a chemical agent or a beverage.

In this embodiment, the electrodes 21 and 22 are formed with flat plates, and these electrodes 21 and 22 are arranged parallel to each other. However, the present invention is not limited thereto, and the electrodes 21 and 22 may also be formed with columnar or ellipse plates, for example. Also, for example, a configuration may also be employed in which the electrodes 21 and 22 are formed with two cylindrical plates having different inner diameters, as in the case of a coaxial cylindrical capacitor, and either one of the electrodes 21 and 22 is arranged within the other one with the center axes thereof matching each other. In this case, an area of the peripheral face of the portion of the electrode 21 that is exposed from the base portion 10, the area opposing the electrode 22, may be covered by the first opposing-side resin portion 31A, and an area of the peripheral face of the portion of the electrode 22 that is exposed from the base portion 10, the area opposing the electrode 21, may be covered by the second opposing-side resin portion 31B. Also, an area of the peripheral face of the portion of the electrode 21 that is exposed from the base portion 10 on the side opposite to the area opposing the electrode 22 may be covered by the first back-side resin portion 32A, and an area of the peripheral face of the portion of the electrode 22 that is exposed from the base portion 10 on the side opposite to the area opposing the electrode 21 may be covered by the second back-side resin portion 32B.

In this embodiment, the connecting resin portion 35 is provided in the resin portion 30A, and the first opposing-side resin portion 31A and the second opposing-side resin portion 31B are connected to each other using the connecting resin portion 35. However, the present invention is not limited thereto, and the connecting resin portion 35 may not be provided in the resin portion 30A.

### Third Embodiment

FIG. 6 is a perspective view of a sensor 1B according to the third embodiment of the present invention. The sensor 1B is provided, in place of the sensor 1 according to the first embodiment of the present invention shown in FIGS. 2 and 3, in the state detection apparatus AA in FIG. 1. This sensor 1B differs from the sensor 1A according to the second embodiment of the present invention shown in FIGS. 4 and 5 in that it includes a resin portion 30B in place of the resin portion 30A, and is the same as the sensor 1A regarding the other portions. Accordingly, the same portions as those in the second embodiment are assigned the same reference numerals, and descriptions thereof are omitted.

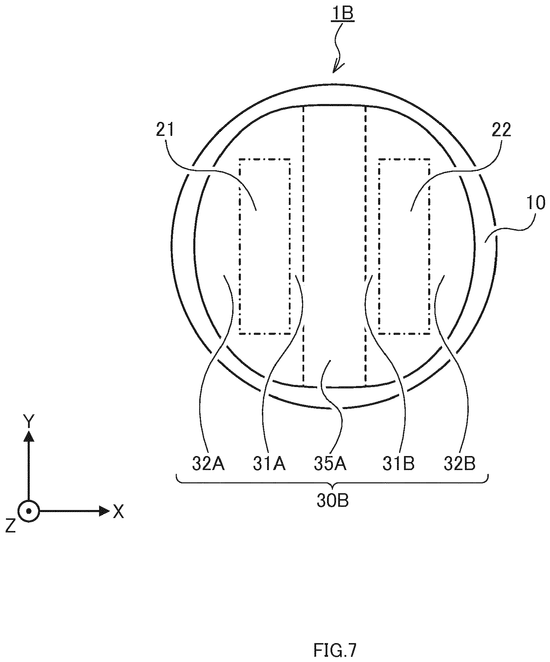

FIG. 7 is a top view of the sensor 1B. The resin portion 30B is made of a non-conductive resin, and is integrated with the electrodes 21 and 22 by means of insertion molding. The resin portion 30B includes a connecting resin portion 35A in addition to the first opposing-side resin portion 31A, the second opposing-side resin portion 31B, the first back-side resin portion 32A, and the second back-side resin portion 32B.

The connecting resin portion 35A connects the first opposing-side resin portion 31A and the second opposing-side resin portion 31B to each other, similarly to the connecting resin portion 35 according to the second embodiment of the present invention. However, the connecting resin portion 35 is in contact with the base portion 10, and connects the first opposing-side resin portion 31A and the second opposing-side resin portion 31B on the base end side (negative Z-side) of the electrodes 21 and 22. On the other hand, the connecting resin portion 35A is spaced apart from the base portion 10, and connects the first opposing-side resin portion 31A and the second opposing-side resin portion 31B to each other on the leading end side (positive Z-side) of the electrodes 21 and 22.

As described above, the sensor 1B includes the first opposing-side resin portion 31A and the second opposing-side resin portion 31B, and the electrodes 21 and 22 are covered by the resin portion 30B by means of insertion molding, similarly to the sensor 1 according to the first embodiment of the present invention. Thus, the sensor 1B can achieve the same effect as that of the sensor 1 according to the first embodiment of the present invention.

Also, similarly to the sensor 1A according to the second embodiment of the present invention, the sensor 1B includes the first back-side resin portion 32A, the second back-side resin portion 32B, and the connecting resin portion 35A that connects the first opposing-side resin portion 31A and the second opposing-side resin portion 31B to each other, and the resin portion 30B is made of a non-conductive resin. Thus, the sensor 1B can achieve the same effect as that of the sensor 1A according to the second embodiment of the present invention.

The electrodes 21 and 22 are embedded, on their base end side, in the base portion 10. Accordingly, the phenomenon that the electrodes 21 and 22 bend due to vibrations of an apparatus to which the sensor 1B is attached or vibrations occurring around this apparatus is more likely to occur on the leading end side of the electrode 21 and 22 than on the base end side. For this reason, in the sensor 1B, the first opposing-side resin portion 31A and the second opposing-side resin portion 31B are connected to each other on the leading end side of the electrodes 21 and 22, using the connecting resin portion 35A. Thus, the bending of the electrodes 21 and 22 due to the aforementioned vibration can be further suppressed.

Note that, in this embodiment, the sensor 1B obtains the permittivity and conductivity of the lubricating oil, and detects the state of the lubricating oil based on the obtained permittivity and conductivity. However, the present invention is not limited thereto, and the sensor 1B may also obtain either one of the permittivity and conductivity of the lubricating oil, and detect the state of the lubricating oil based on the obtained result. Alternatively, the sensor 1B may also obtain an electrical characteristic of the lubricating oil other than the permittivity and conductivity (e.g. capacitance, dissipation factor (tanδ), characteristic impedance Z etc.), and detect the state of the lubricating oil based on the obtained electrical characteristic.

In this embodiment, the sensor 1B detects the state of the lubricating oil. However, the present invention is not limited thereto, and the state of any kind of fluid can be detected. For example, the sensor 1B can also detect the degree of contamination of water, and can also detect the quality of a chemical agent or a beverage.

In this embodiment, the electrodes 21 and 22 are formed with flat plates, and these electrodes 21 and 22 are arranged parallel to each other. However, the present invention is not limited thereto, and the electrodes 21 and 22 may also be formed with columnar or ellipse plates, for example. Also, for example, a configuration may also be employed in which the electrodes 21 and 22 are formed with two cylindrical plates having different inner diameters, as in the case of a coaxial cylindrical capacitor, and either one of the electrodes 21 and 22 is arranged within the other one with the center axes thereof matching each other. In this case, an area of the peripheral face of the portion of the electrode 21 that is exposed from the base portion 10, the area opposing the electrode 22, may be covered by the first opposing-side resin portion 31A, and an area of the peripheral face of the portion of the electrode 22 that is exposed from the base portion 10, the area opposing the electrode 21, may be covered by the second opposing-side resin portion 31B. Also, an area of the peripheral face of the portion of the electrode 21 that is exposed from the base portion 10 on the side opposite to the area opposing the electrode 22 may be covered by the first back-side resin portion 32A, and an area of the peripheral face of the portion of the electrode 22 that is exposed from the base portion 10 on the side opposite to the area opposing the electrode 21 may be covered by the second back-side resin portion 32B.

In this embodiment, the connecting resin portion 35A is provided in the resin portion 30B, and the first opposing-side resin portion 31A and the second opposing-side resin portion 31B are connected to each other on the leading end side of the electrodes 21 and 22, using the connecting resin portion 35A. However, a configuration may also be employed in which the connecting resin portion 35 according to the second embodiment of the present invention shown in FIG. 4 is also provided in the resin portion 30B, and the first opposing-side resin portion 31A and the second opposing-side resin portion 31B are also connected to each other on the base end side of the electrodes 21 and 22.

### Fourth Embodiment

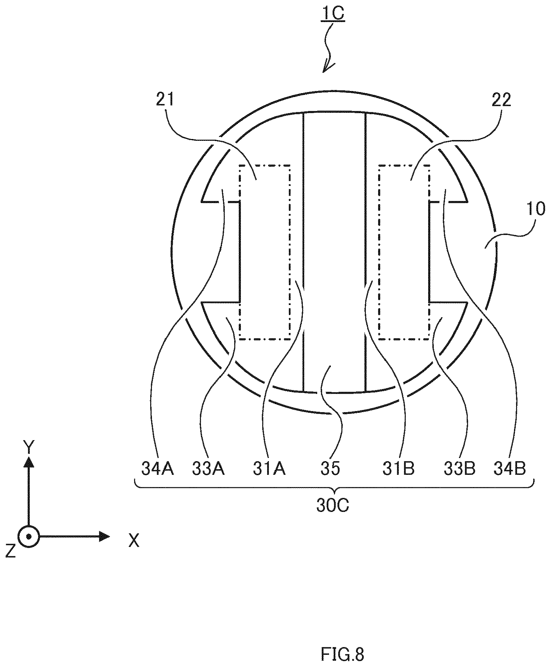

FIG. 8 is a top view of a sensor 1C according to the fourth embodiment of the present invention. The sensor 1C is provided, in place of the sensor 1 according to the first embodiment of the present invention shown in FIGS. 2 and 3, in the state detection apparatus AA in FIG. 1. This sensor 1C differs from the sensor 1A according to the second embodiment of the present invention shown in FIGS. 4 and 5 in that it includes a resin portion 30C in place of the resin portion 30A, and is the same as the sensor 1A regarding the other portions. Accordingly, the same portions as those in the second embodiment are assigned the same reference numerals, and descriptions thereof are omitted.

The resin portion 30C is made of a non-conductive resin, and is integrated with the electrodes 21 and 22 by means of insertion molding. The resin portion 30C includes first back-side resin portions 33A and 34A and second back-side resin portions 33B and 34B in addition to the first opposing-side resin portion 31A, the second opposing-side resin portion 31B, and the connecting resin portion 35.

The first back-side resin portions 33A and 34A extend in the length direction (Z direction) of the electrode 21, and are formed in a rib shape and spaced apart from each other in the width direction (Y direction) of the electrode 21. One end side (negative Y-side) of the electrode 21 in the width direction of the electrode 21 is covered by the first back-side resin portion 33A, and the other end side (positive Y-side) of the electrode 21 in the width direction of the electrode 21 is covered by the first back-side resin portion 34A. Meanwhile, the center portion of the electrode 21 in the width direction of the electrode 21 is not covered by the first back-side resin portion 33A or 34A, and is exposed.

The second back-side resin portions 33B and 34B extend in the length direction (Z direction) of the electrode 22, and are formed in a rib shape and spaced apart from each other in the width direction (Y direction) of the electrode 22. One end side (negative Y-side) of the electrode 22 in the width direction of the electrode 22 is covered by the second back-side resin portion 33B, and the other end side (positive Y-side) of the electrode 22 in the width direction of the electrode 22 is covered by the second back-side resin portion 34B. Meanwhile, the center portion of the electrode 22 in the width direction of the electrode 22 is not covered by the second back-side resin portion 33B or 34B, and is exposed.

As described above, the sensor 1C includes the first opposing-side resin portion 31A and the second opposing-side resin portion 31B, and the electrodes 21 and 22 are covered by the resin portion 30B by means of insertion molding, similarly to the sensor 1 according to the first embodiment of the present invention. Thus, the sensor 1C can achieve the same effect as that of the sensor 1 according to the first embodiment of the present invention.

Also, the sensor 1C includes the first back-side resin portions 33A and 34A, the second back-side resin portions 33B and 34B, and the connecting resin portion 35 that connects the first opposing-side resin portion 31A and the second opposing-side resin portion 31B to each other, similarly to the sensor 1A according to the second embodiment of the present invention, and the resin portion 30C is made of a non-conductive resin. Thus, the sensor 1C can achieve the same effect as that of the sensor 1A according to the second embodiment of the present invention.

In the sensor 1C, the center portion of the electrode 21 in the width direction of the electrode 21 is not covered by the first back-side resin portion 33A or 34A and is exposed, and the center portion of the electrode 22 in the width direction of the electrode 22 is not covered by the second back-side resin portion 33B or 34B and is exposed. Thus, the amount of resin required for the resin portion 30C can be reduced while increasing the rigidity of the electrode 21 in the thickness direction of the electrode 21 using the first back-side resin portions 33A and 34A, and increasing the rigidity of the electrode 22 in the thickness direction of the electrode 22 using the second back-side resin portions 33B and 34B.

Although, in this embodiment, the resin portion 30C is made of a non-conductive resin, the present invention is not limited thereto, and the resin portion 30C may also be made of a conductive resin. However, in the case of making the resin portion 30C using a conductive resin, a configuration in which the connecting resin portion 35 is not provided in the resin portion 30C needs to be employed.

In this embodiment, the sensor 1C obtains the permittivity and conductivity of the lubricating oil, and detects the state of the lubricating oil based on the obtained permittivity and conductivity. However, the present invention is not limited thereto, and the sensor 1C may also obtain either one of the permittivity and conductivity of the lubricating oil, and detect the state of the lubricating oil based on the obtained result. Alternatively, the sensor 1C may also obtain an electrical characteristic of the lubricating oil other than the permittivity and conductivity (e.g. capacitance, dissipation factor (tanδ), characteristic impedance Z etc.), and detect the state of the lubricating oil based on the obtained electrical characteristic.

In this embodiment, the sensor 1C detects the state of the lubricating oil. However, the present invention is not limited thereto, and the state of any kind of fluid can be detected. For example, the sensor 1C can also detect the degree of contamination of water, and can also detect the quality of a chemical agent or a beverage.

In this embodiment, the electrodes 21 and 22 are formed with flat plates, and these electrodes 21 and 22 are arranged parallel to each other. However, the present invention is not limited thereto, and the electrodes 21 and 22 may also be formed with columnar or ellipse plates, for example. Also, for example, a configuration may also be employed in which the electrodes 21 and 22 are formed with two cylindrical plates having different inner diameters, as in the case of a coaxial cylindrical capacitor, and either one of the electrodes 21 and 22 is arranged within the other one with the center axes thereof matching each other. In this case, an area of the peripheral face of the portion of the electrode 21 that is exposed from the base portion 10, the area opposing the electrode 22, may be covered by the first opposing-side resin portion 31A, and an area of the peripheral face of the portion of the electrode 22 that is exposed from the base portion 10, the area opposing the electrode 21, may be covered by the second opposing-side resin portion 31B. Also, an area of the peripheral face of the portion of the electrode 21 that is exposed from the base portion 10 on the side opposite to the area opposing the electrode 22, may be covered by the first back-side resin portions 33A and 34A, and an area of the peripheral face of the portion of the electrode 22 that is exposed from the base portion 10 on the side opposite to the area opposing the electrode 21, may be covered by the second back-side resin portions 33B and 34B.

In this embodiment, two first back-side resin portions, namely the first back-side resin portions 33A and 34A are formed in a rib shape. However, the present invention is not limited thereto, and only one of them may also be formed in a rib shape, or three of them may also be formed in a rib shape. In this embodiment, two second back-side resin portions, namely the second back-side resin portions 33B and 34B are formed in a rib shape. However, the present invention is not limited thereto, and only one of them may also be formed in a rib shape, or three of them may also be formed in a rib shape.

In this embodiment, the center portion of the electrode 21 in the width direction of the electrode 21 is not covered by the first back-side resin portion 33A or 34A and is exposed. However, the present invention is not limited thereto. A configuration may also be employed in which a connecting portion that is formed so as to be thinner than the thickness of the first back-side resin portions 33A and 34A in the X direction is provided, and the center portion of the electrode 21 in the width direction of the electrode 21 is covered by this connecting portion. Also, in this embodiment, the center portion of the electrode 22 in the width direction of the electrode 22 is not covered by the second back-side resin portion 33B or 34B and is exposed. However, the present invention is not limited thereto. A configuration may also be employed in which a connecting portion that is formed so as to be thinner than the thickness of the second back-side resin portions 33B and 34B in the X direction is provided, and the center portion of the electrode 22 in the width direction of the electrode 22 is covered by this connecting portion.

In this embodiment, the connecting resin portion 35 is provided in the resin portion 30C, and the first opposing-side resin portion 31A and the second opposing-side resin portion 31B are connected to each other using the connecting resin portion 35. However, the present invention is not limited thereto, and the connecting resin portion 35 may not be provided in the resin portion 30C.

The first to fourth embodiments have been described above. These embodiments have the effect of more appropriately detecting the state of a fluid.

Note that, although the embodiments of this invention have been described in detail with reference to the drawings, the specific configuration is not limited to these embodiments, and also includes any design that does not depart from the gist of the invention, for example.

Furthermore, the present invention is also applicable to water hydraulic equipment, for example.

### Reference Signs List

AA State detection apparatus
1, 1A, 1B, 1C Sensor
2Feature amount acquisition unit
3Detection unit
10 Base Portion
21,22 Electrode
30,30A,30B,30C Resin portion
31A First opposing-side resin portion
31B Second opposing-side resin portion
32A, 33A, 34A First back-side resin portion
32B, 33B, 34B Second back-side resin portion
35, 35A Connecting resin portion

## Claims

1. A sensor for detecting a state of a fluid, comprising:
a first electrode and a second electrode that are arranged parallel to each other;
a first opposing-side resin portion that covers an area of a peripheral face of the first electrode, the area opposing the second electrode; and
a second opposing-side resin portion that covers an area of a peripheral face of the second electrode, the area opposing the first electrode,
wherein the first opposing-side resin portion and the second opposing-side resin portion are spaced apart from each other.

2. The sensor according to claim 1, further comprising:
a first back-side resin portion that covers at least a portion of an area of the peripheral face of the first electrode on a side opposite to the area opposing the second electrode; and
a second back-side resin portion that covers at least a portion of an area of the peripheral face of the second electrode on a side opposite to the area opposing the first electrode.

3. The sensor according to claim 1, further comprising:
a connecting resin portion that connects the first opposing-side resin portion and the second opposing-side resin portion to each other at at least one of one end and the other end of the electrodes in their length direction.

4. The sensor according to claim 3,
wherein the connecting resin portion is made of a non-conductive resin.

5. A method for manufacturing a sensor for detecting a state of a fluid, the method comprising:
a step of arranging a first electrode and a second electrode parallel to each other; and
a step of covering, with resin, an area of a peripheral face of the first electrode, the area opposing the second electrode, and an area of a peripheral phase of the second electrode, the area opposing the first electrode by means of insertion molding, and forming a first opposing-side resin portion for covering the area of the peripheral face of the first electrode, the area opposing the second electrode, and a second opposing-side resin portion for covering the area of the peripheral face of the second electrode, the area opposing the first electrode, so that the first opposing-side resin portion and the second opposing-side resin portion are spaced apart from each other.
